# EUROPÄISCHE PATENTANMELDUNG

(11) **EP 1 064 878 A2**
(43) Veröffentlichungstag der Anmeldung: **03.01.2001**
(21) Anmeldenummer: 00113658.9
(22) Anmeldetag: 28.06.2000
(51) Int. Cl.: A61B 1/267

(54) **Hilfsvorrichtung zum Einführen von Beatmungstuben in den Mundraum eines Patienten**

(30) Priorität: 02.07.1999 DE 29911559 U
(71) Anmelder: Schlüren, Markus, 72764 Reutlingen (DE)
(72) Erfinder: Schlüren, Markus, 72764 Reutlingen (DE)
(74) Vertreter: Möbus, Rudolf, Dipl.-Ing.

(57) **Zusammenfassung**

Eine Hilfsvorrichtung zum Einführen von Beatmungstuben in den Mundraum eines Patienten, insbesondere ein Laryngoskop-Spatel mit einem Handgriff (11) und einem daran lösbar angeordneten Spatel (10), wobei der Spatel (10) mehrere ineinander eingesetzte und teleskopartig ausziehbare Elemente (12, 13) aufweist.

## Beschreibung

Die Erfindung betrifft eine Hilfsvorrichtung zum Einführen von Beatmungstuben in den Mundraum eines Patienten, insbesondere einen Laryngoskop-Spatel mit einem Handgriff und einem daran lösbar angeordneten Spatel.

Bisher gibt es Laryngoskop-Spatel in verschiedenen Größen, je nach der Körpergröße des Patienten. Dies führt dazu, dass beispielsweise Rettungssanitäter immer einen Rettungskoffer mit einem Satz unterschiedlich großer Spatel mit sich führen müssen. Dies ist sehr unpraktisch, da dieser Spatelsatz relativ viel Platz benötigt und für den Träger außerdem ein zusätzliches Gewicht im Rettungskoffer darstellt.

Die Erfindung hat die Aufgabe, eine eingangs genannte Hilfsvorrichtung dahin gehend zu verbessern, dass diese Platz- und Gewichtsprobleme vermieden werden können.

Die Erfindung löst die gestellte Aufgabe mittels einer Hilfsvorrichtung zum Einführen von Beatmungstuben in den Mundraum eines Patienten, insbesondere einem Laryngoskop-Spatel mit einem Handgriff und einem daran lösbar angeordneten Spatel, wobei der Spatel mehrere ineinander eingesetzte und teleskopartig ausziehbare Elemente aufweist. Dieser Spatel ist durch entsprechende Einstellung für Patienten verschiedener Körpergröße einsetzbar. Folglich muss beim Rettungseinsatz nur noch ein deutlich kleinerer Spatelsatz mitgeführt werden als seither, sodass zukünftig mehr Platz im Rettungskoffer ist und der Träger des Rettungskoffers nicht mehr so schwer tragen muss.

Damit der Spatel bei Patienten unterschiedlicher Körpergröße gleichermaßen sicher einsetzbar ist, können die Elemente im Gebrauch in den verschiedenen Ausziehstufen eingerastet werden.

Zum schnellen Zusammenbauen und Zerlegen kann die Hilfsvorrichtung eine Rastverbindung zwischen dem Handgriff und dem Spatel aufweisen. Dies ermöglicht insbesondere die rasche Demontage des Spatels nach Gebrauch zu Sterilisationszwecken. Besonders vorteilhaft ist es dabei, wenn der Spatel autoklavierbar ist.

Entsprechend der schon bewährten Formen eines Foregger- oder McIntosh-Spatels kann der Spatel der Hilfsvorrichtung eine dieser beiden Formen aufweisen.

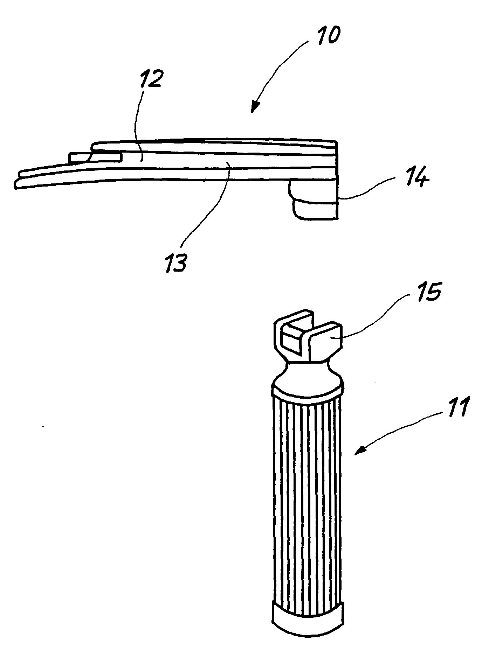

Nachfolgend wird ein Ausführungsbeispiel einer erfindungsgemäßen Hilfsvorrichtung anhand der beiliegenden Zeichnung näher erläutert.

Die einzige Figur zeigt eine erfindungsgemäße Hilfsvorrichtung mit einem Spatel 10 und einem Handgriff 11. Der Spatel 10 weist zwei ineinander eingesetzte und teleskopartig ausziehbare Elemente 12 und 13 auf. Je nach Körpergröße des Patienten kann das Element 12 mehr oder weniger oder auch gar nicht aus dem Element 13 herausgezogen werden. Dabei ist das Element 12 in jeder Ausziehstufe einrastbar. Der Spatel 10 weist ein Kupplungsstück 14 auf, das mit einem Kupplungsstück 15 des Handgriffs 11 verrastet werden kann. Somit können der Spatel 10 und der Handgriff 11 bei einem Einsatz zusammengesteckt und nach dem Einsatz die Rastverbindung durch Lösen der Kupplungsstücke 14 und 15 wieder getrennt werden. Dadurch lassen sich der Spatel 10 und der Handgriff 11 auf engem Raum gut verstauen.

## Patentansprüche

1. Hilfsvorrichtung zum Einführen von Beatmungstuben in den Mundraum eines Patienten, insbesondere ein Laryngoskop-Spatel mit einem Handgriff (11) und einem daran lösbar angeordneten Spatel (10), dadurch gekennzeichnet, dass der Spatel (10) mehrere ineinander eingesetzte und teleskopartig ausziehbare Elemente (12, 13) aufweist.

2. Hilfsvorrichtung nach Anspruch 1, dadurch gekennzeichnet, dass die Elemente (12, 13) in verschiedenen Ausziehstufen einrastbar sind.

3. Hilfsvorrichtung nach Anspruch 1 oder 2, dadurch gekennzeichnet, dass sie eine Rastverbindung zwischen dem Handgriff (11) und dem Spatel (10) aufweist.

4. Hilfsvorrichtung nach einem der Ansprüche 1 bis 3, dadurch gekennzeichnet, dass sie autoklavierbar ist.

5. Hilfsvorrichtung nach einem der Ansprüche 1 bis 4, dadurch gekennzeichnet, dass der Spatel (10) in der Form einem Foregger- oder McIntosh-Spatel entspricht.
